# EUROPEAN PATENT APPLICATION

(11) **EP 4 462 321 A1**
(43) Date of publication of application: **13.11.2024**
(21) Application number: 24159568.5
(22) Date of filing: 26.02.2024
(51) Int. Cl.: G06N 20/20

(54) **INFORMATION PROCESSING DEVICE, INFORMATION PROCESSING SYSTEM, AND INFORMATION PROCESSING METHOD**

(30) Priority: 12.05.2023 JP 2023079271
(71) Applicant: Hitachi, Ltd., Tokyo 100-8280 (JP)
(72) Inventor: CHALKIDIS, George, Chiyoda-ku,, 100-8280 (JP); TAKEUCHI, Wataru, Chiyoda-ku,, 100-8280 (JP)
(74) Representative: Strehl Schübel-Hopf & Partner

(57) **Abstract**

Aspects relate to providing an information processing technique for generating machine learning models with a high degree of robustness that are capable of producing reliable results for data sets having small sample sizes. An information processing device includes a variance analysis unit for generating analysis results for an original dataset and identifying a first set of subjects from among the original dataset associated with a first analysis result that achieves a variance threshold; a partition unit for partitioning the first set of subjects into original data partitions and generating copied data partitions; a modification unit for generating modified copy data partitions; and a result generation unit for training machine learning models using the original data partitions and the modified copy data partitions, generating a second analysis result using the machine learning models, and generating a final analysis result by aggregating the first analysis result and the second analysis result.

## Description

### [Technical Field]

The present disclosure relates to an information processing device, an information processing method and an information processing system.

### [Background Art]

In recent years, machine learning techniques have been developed for application to a wide range of fields. In machine learning, training data based on known cases is input to a computer. The computer analyzes the training data to learn a model that generalizes the relationship between factors (sometimes called explanatory variables or independent variables) and outcomes (sometimes called objective variables or dependent variables). This model can then be used to predict results for unknown cases. As an example, it is possible to generate a model that predicts the survivability of patients subjected to different medical interventions based on data including past medical intervention history and patient characteristics for similar patients.

Conventionally, techniques for improving the performance of machine learning techniques have been considered.

For example, US Patent 9600231B1 (Patent Document 1) discloses a technique in which "A revised support vector machine (SVM) classifier is offered to distinguish between true keywords and false positives based on output from a keyword spotting component of a speech recognition system. The SVM operates on a reduced set of feature dimensions, where the feature dimensions are selected based on their ability to distinguish between true keywords and false positives. Further, support vectors pairs are merged to create a reduced set of re-weighted support vectors. These techniques result in an SVM that may be operated using reduced computing resources, thus improving system performance."

### [Citation List]

### [Patent Literature]

### [PTL 1]

US9600231B1

### [Summary of Invention]

### [Technical Problem]

In machine learning, it is preferable that the accuracy of a generated model, that is, the ability to accurately predict the result of an unknown case (sometimes called prediction performance) is high. The prediction performance increases as the learnable information content in a dataset for a model to generalize relationships between factors increases, which is often attainable with larger sample sizes. Conversely, datasets based on small sample sizes can lead to decreased prediction performance, such as results with large variability in outcomes. Due to this high variance between different outcomes, such results may be unreliable, and are not suitable for drawing conclusions or insights.

Patent Document 1 proposes a technique for using an SVM classifier for keyword recognition. More particularly, the technique of Patent Document 1 relates to reducing the size of a feature set in order to facilitate operation of the SVM in environments with limited computing resources. However, Patent Document 1 does not relate to improving prediction performance of machine learning models on data sets with small sample sizes.

Accordingly, it is an object of the present disclosure to provide an information processing technique for generating machine learning models with a high degree of robustness that are capable of producing reliable results for data sets having small sample sizes.

### [Solution to Problem]

One representative example of the present disclosure relates to an information processing device including a processor; and a memory containing computer implementable instructions executable by the processor to cause the processor to function as: a variance analysis unit configured to: generate, by processing an original dataset with a first set of machine learning models, a set of analysis results, and identify, based on the set of analysis results, a high-variance subset from the original dataset that includes a first set of subjects associated with a first analysis result that achieves a variance threshold; a partition unit configured to: partition the first set of subjects of the high-variance subset into a first data partition and a second data partition, and generate a third data partition that is a copy of the first data partition and a fourth data partition that is a copy of the second data partition, a modification unit configured to generate a modified third data partition by modifying the third data partition and a modified fourth data partition by modifying the fourth data partition; and a result generation unit configured to: train a second set of machine learning models using the first data partition, the second data partition, the modified third data partition and the modified fourth data partition, generate, by processing the original dataset with the second set of machine learning models, a second analysis result, and generate a final analysis result for the first set of subjects of the high-variance subset by aggregating and classifying the first analysis result and the second analysis result.

### [Advantageous Effects of Invention]

According to the present disclosure it is possible to provide an information processing technique for generating machine learning models with a high degree of robustness that are capable of producing reliable results for data sets having small sample sizes.

Problems, configurations, and effects other than those described above will be made clear by the following description in the embodiments for carrying out the invention.

### [Brief Description of Drawings]

[Fig. 1]

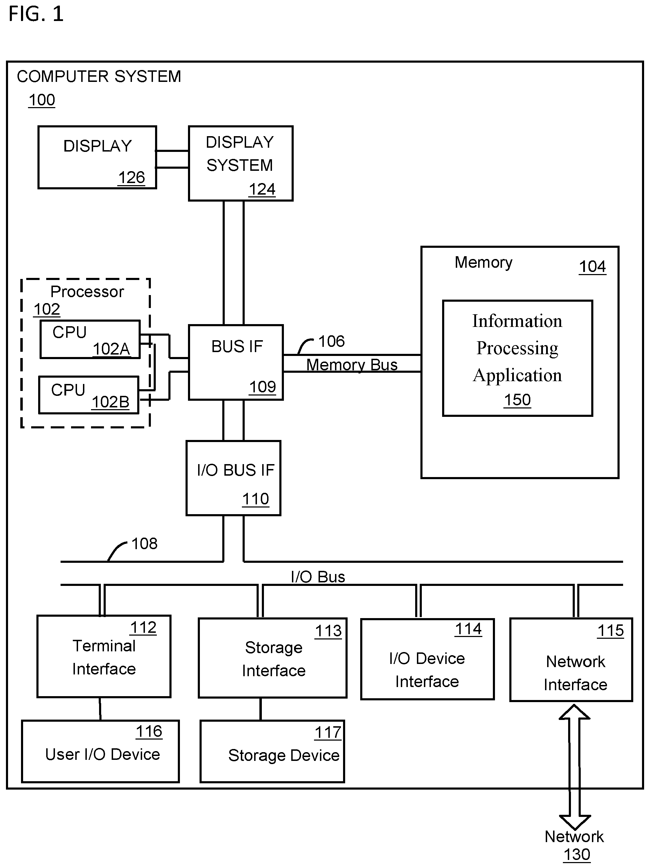

   FIG. 1 illustrates an example computing architecture for executing the embodiments of the present disclosure.
[Fig. 2]

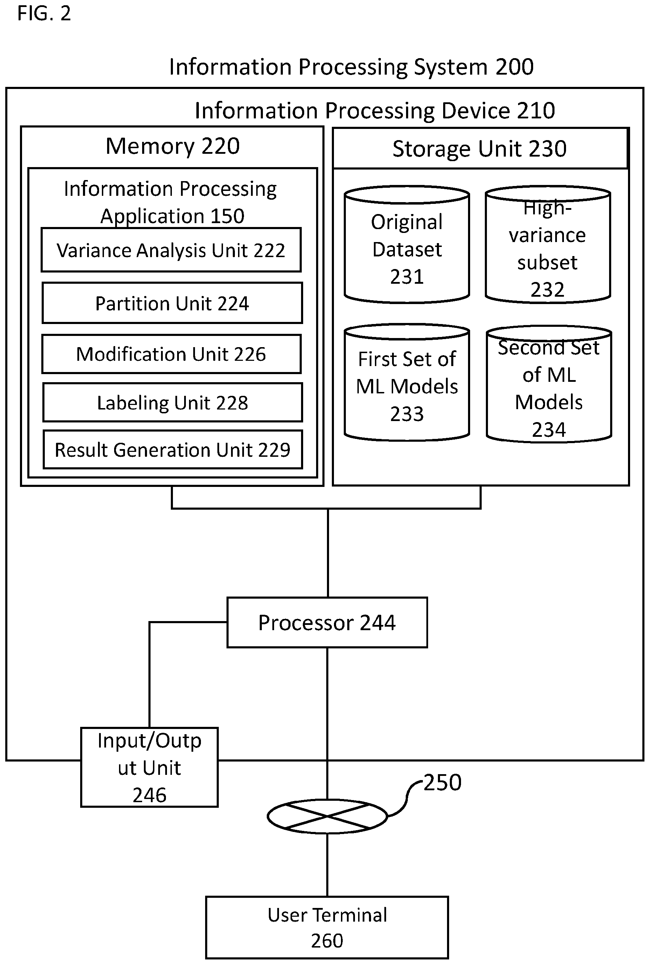

   FIG. 2 is a diagram illustrating an example hardware configuration of an information processing system according to the embodiments of the present disclosure.
[Fig. 3]

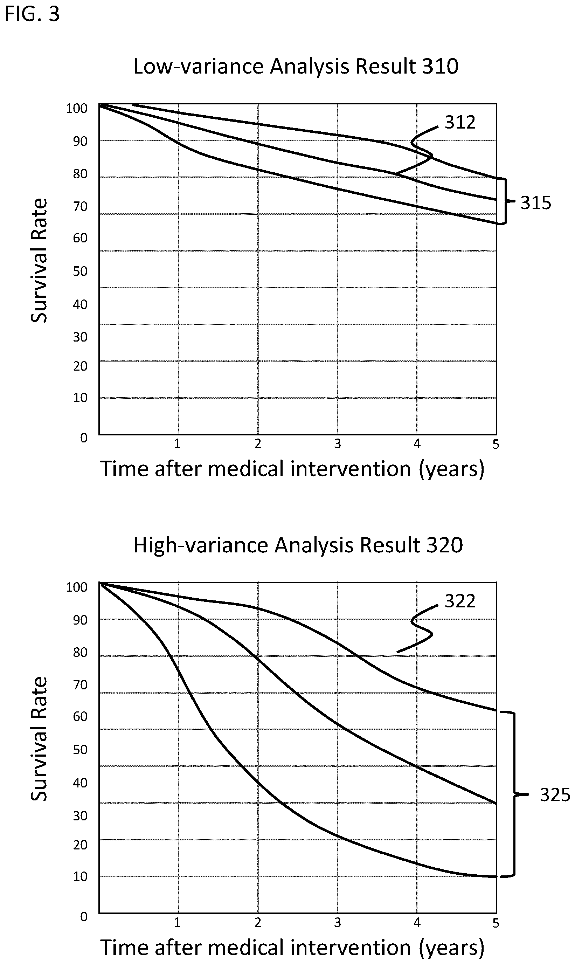

   FIG. 3 is a diagram illustrating an example of a low-variance analysis result and a high-variance analysis result, according to the embodiments of the present disclosure.
[Fig. 4]

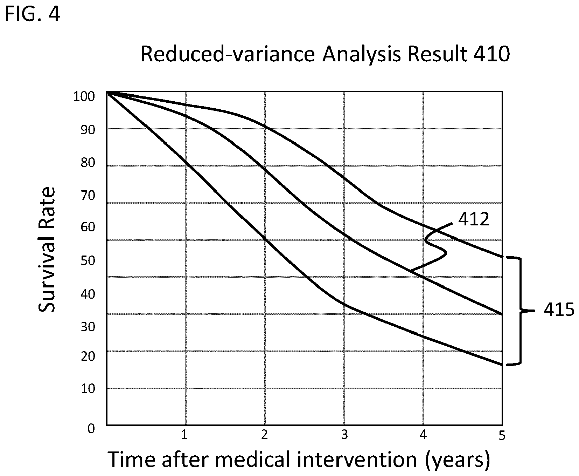

   FIG. 4 is a diagram illustrating an example of a reduced-variance analysis result according to the embodiments of the present disclosure.
[Fig. 5]

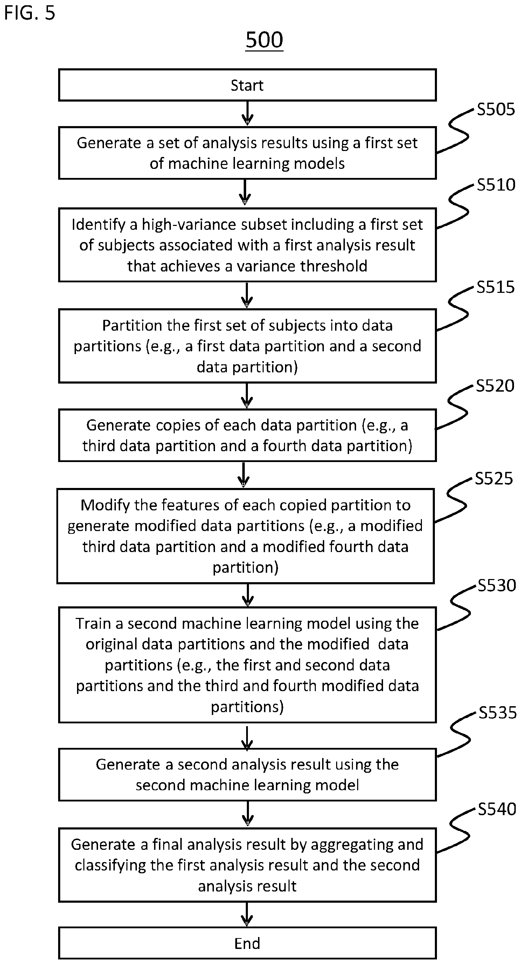

   FIG. 5 is a flowchart illustrating an information processing method according to the embodiments of the present disclosure.
[Fig. 6]

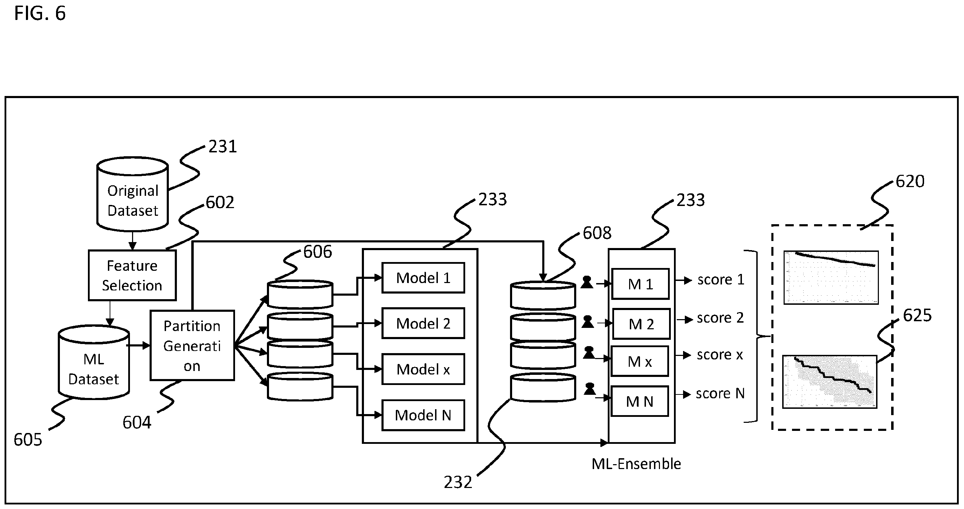

   FIG. 6 is a diagram illustrating an example logical configuration of an information processing system for identifying datasets associated with high variance results, according to the embodiments of the present disclosure.
[Fig. 7]

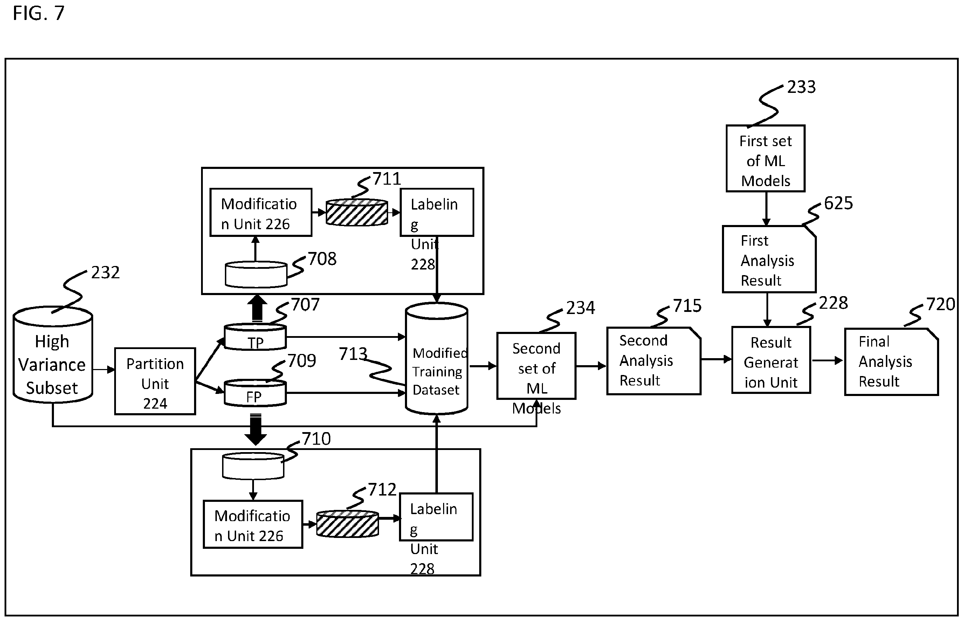

   FIG. 7 is a diagram illustrating an example logical configuration of an information processing system for generating analysis results for high-variance datasets, according to the embodiments of the present disclosure.

### [Description of Embodiment(s)]

Herein, embodiments of the present invention will be described with reference to the Figures. It should be noted that the embodiments described herein are not intended to limit the invention according to the claims, and it is to be understood that each of the elements and combinations thereof described with respect to the embodiments are not strictly necessary to implement the aspects of the present invention.

Various aspects are disclosed in the following description and related drawings. Alternate aspects may be devised without departing from the scope of the disclosure. Additionally, well-known elements of the disclosure will not be described in detail or will be omitted so as not to obscure the relevant details of the disclosure.

The words "exemplary" and/or "example" are used herein to mean "serving as an example, instance, or illustration." Any aspect described herein as "exemplary" and/or "example" is not necessarily to be construed as preferred or advantageous over other aspects. Likewise, the term "aspects of the disclosure" does not require that all aspects of the disclosure include the discussed feature, advantage or mode of operation.

Further, many aspects are described in terms of sequences of actions to be performed by, for example, elements of a computing device. It will be recognized that various actions described herein can be performed by specific circuits (e.g., an application specific integrated circuit (ASIC)), by program instructions being executed by one or more processors, or by a combination of both. Additionally, the sequence of actions described herein can be considered to be embodied entirely within any form of computer readable storage medium having stored therein a corresponding set of computer instructions that upon execution would cause an associated processor to perform the functionality described herein. Thus, the various aspects of the disclosure may be embodied in a number of different forms, all of which have been contemplated to be within the scope of the claimed subject matter.

Hereinafter, a detailed description of the embodiments of the present disclosure will be described with reference to the Figures.

Turning now to the Figures, FIG. 1 depicts a high-level block diagram of a computer system 100 for implementing various embodiments of the present disclosure, according to embodiments. The mechanisms and apparatus of the various embodiments disclosed herein apply equally to any appropriate computing system. The major components of the computer system 100 include one or more processors 102, a memory 104, a terminal interface 112, a storage interface 113, an I/O (Input/Output) device interface 114, and a network interface 115, all of which are communicatively coupled, directly or indirectly, for inter-component communication via a memory bus 106, an I/O bus 108, bus interface unit 109, and an I/O bus interface unit 110.

The computer system 100 may contain one or more general-purpose programmable central processing units (CPUs) 102A and 102B, herein generically referred to as the processor 102. In embodiments, the computer system 100 may contain multiple processors; however, in certain embodiments, the computer system 100 may alternatively be a single CPU system. Each processor 102 executes instructions stored in the memory 104 and may include one or more levels of on-board cache.

In embodiments, the memory 104 may include a random-access semiconductor memory, storage device, or storage medium (either volatile or non-volatile) for storing or encoding data and programs. In certain embodiments, the memory 104 represents the entire virtual memory of the computer system 100, and may also include the virtual memory of other computer systems coupled to the computer system 100 or connected via a network. The memory 104 can be conceptually viewed as a single monolithic entity, but in other embodiments the memory 104 is a more complex arrangement, such as a hierarchy of caches and other memory devices. For example, memory may exist in multiple levels of caches, and these caches may be further divided by function, so that one cache holds instructions while another holds non-instruction data, which is used by the processor or processors. Memory may be further distributed and associated with different CPUs or sets of CPUs, as is known in any of various so-called non-uniform memory access (NUMA) computer architectures.

The memory 104 may store all or a portion of the various programs, modules and data structures for processing data transfers as discussed herein. For instance, the memory 104 can store an information processing application 150. In embodiments, the information processing application 150 may include instructions or statements that execute on the processor 102 or instructions or statements that are interpreted by instructions or statements that execute on the processor 102 to carry out the functions as further described below.

In certain embodiments, the information processing application 150 is implemented in hardware via semiconductor devices, chips, logical gates, circuits, circuit cards, and/or other physical hardware devices in lieu of, or in addition to, a processor-based system. In embodiments, the information processing application 150 may include data in addition to instructions or statements. In certain embodiments, a camera, sensor, or other data input device (not shown) may be provided in direct communication with the bus interface unit 109, the processor 102, or other hardware of the computer system 100. In such a configuration, the need for the processor 102 to access the memory 104 and the information processing application 150 may be reduced.

The computer system 100 may include a bus interface unit 109 to handle communications among the processor 102, the memory 104, a display system 124, and the I/O bus interface unit 110. The I/O bus interface unit 110 may be coupled with the I/O bus 108 for transferring data to and from the various I/O units. The I/O bus interface unit 110 communicates with multiple I/O interface units 112, 113, 114, and 115, which are also known as I/O processors (IOPs) or I/O adapters (IOAs), through the I/O bus 108. The display system 124 may include a display controller, a display memory, or both. The display controller may provide video, audio, or both types of data to a display device 126. Further, the computer system 100 may include one or more sensors or other devices configured to collect and provide data to the processor 102.

As examples, the computer system 100 may include biometric sensors (e.g., to collect heart rate data, stress level data), environmental sensors (e.g., to collect humidity data, temperature data, pressure data), motion sensors (e.g., to collect acceleration data, movement data), or the like. Other types of sensors are also possible. The display memory may be a dedicated memory for buffering video data. The display system 124 may be coupled with a display device 126, such as a standalone display screen, computer monitor, television, or a tablet or handheld device display.

In one embodiment, the display device 126 may include one or more speakers for rendering audio. Alternatively, one or more speakers for rendering audio may be coupled with an I/O interface unit. In alternate embodiments, one or more of the functions provided by the display system 124 may be on board an integrated circuit that also includes the processor 102. In addition, one or more of the functions provided by the bus interface unit 109 may be on board an integrated circuit that also includes the processor 102.

The I/O interface units support communication with a variety of storage and I/O devices. For example, the terminal interface unit 112 supports the attachment of one or more user I/O devices 116, which may include user output devices (such as a video display device, speaker, and/or television set) and user input devices (such as a keyboard, mouse, keypad, touchpad, trackball, buttons, light pen, or other pointing device). A user may manipulate the user input devices using a user interface in order to provide input data and commands to the user I/O device 116 and the computer system 100, and may receive output data via the user output devices. For example, a user interface may be presented via the user I/O device 116, such as displayed on a display device, played via a speaker, or printed via a printer.

The storage interface 113 supports the attachment of one or more disk drives or direct access storage devices 117 (which are typically rotating magnetic disk drive storage devices, although they could alternatively be other storage devices, including arrays of disk drives configured to appear as a single large storage device to a host computer, or solid-state drives, such as flash memory). In some embodiments, the storage device 117 may be implemented via any type of secondary storage device. The contents of the memory 104, or any portion thereof, may be stored to and retrieved from the storage device 117 as needed. The I/O device interface 114 provides an interface to any of various other I/O devices or devices of other types, such as printers or fax machines. The network interface 115 provides one or more communication paths from the computer system 100 to other digital devices and computer systems; these communication paths may include, for example, one or more networks 130.

Although the computer system 100 shown in FIG. 1 illustrates a particular bus structure providing a direct communication path among the processors 102, the memory 104, the bus interface 109, the display system 124, and the I/O bus interface unit 110, in alternative embodiments the computer system 100 may include different buses or communication paths, which may be arranged in any of various forms, such as point-to-point links in hierarchical, star or web configurations, multiple hierarchical buses, parallel and redundant paths, or any other appropriate type of configuration. Furthermore, while the I/O bus interface unit 110 and the I/O bus 108 are shown as single respective units, the computer system 100 may, in fact, contain multiple I/O bus interface units 110 and/or multiple I/O buses 108. While multiple I/O interface units are shown which separate the I/O bus 108 from various communications paths running to the various I/O devices, in other embodiments, some or all of the I/O devices are connected directly to one or more system I/O buses.

In various embodiments, the computer system 100 is a multi-user mainframe computer system, a single-user system, or a server computer or similar device that has little or no direct user interface, but receives requests from other computer systems (clients). In other embodiments, the computer system 100 may be implemented as a desktop computer, portable computer, laptop or notebook computer, tablet computer, pocket computer, telephone, smart phone, or any other suitable type of electronic device.

Next, with reference to FIG. 2, an example hardware configuration of an information processing system according to the embodiments of the present disclosure will be described.

FIG. 2 is a diagram illustrating an example hardware configuration of an information processing system 200 according to the embodiments of the present disclosure. The information processing system 200 relates to an information processing system configured to identify datasets associated with high-variance analysis results and generate machine learning models with a high degree of robustness that are capable of producing reliable results for data sets having small sample sizes.

As illustrated in FIG. 2, the information processing system 200 according to the embodiments of the present disclosure includes an information processing device 210, a communication network 250, and a user terminal 260. The information processing device 210 and the user terminal 260 may be communicatively connected via the communication network 250.

Here, the communication network 250 may include a Local Area Network (LAN) connection, the Internet, a Wide Area Network (WAN) connection, a Metropolitan Area Network (MAN) connection or the like.

The information processing device 210 is an apparatus for identifying datasets associated with high-variance analysis results and generating machine learning models with a high degree of robustness that are capable of producing reliable results for data sets having small sample sizes, and as illustrated in FIG. 2, primarily includes a memory 220, a storage unit 230, a processor 244, and an input/output unit 246. In embodiments, the information processing device 210 may be implemented using a computer system such as the computer system 100 illustrated in FIG. 1.

The memory 220 is a memory for storing an information processing application 150 for implementing the functions of the information processing technique according to the embodiments of the present disclosure. As illustrated in FIG. 2, the information processing application 150 may include a variance analysis unit 222, a partition unit 224, a modification unit 226, a labeling unit 228 and a result generation unit 229. Each of the variance analysis unit 222, the partition unit 224, the modification unit 226, the labeling unit 228 and the result generation unit 229 may be implemented as software modules comprising the information processing application 150.

The variance analysis unit 222 is a functional unit for identifying datasets associated with high-variance analysis results. More particularly, the variance analysis unit 222 may generate, by processing an original dataset with a first set of machine learning models (e.g., the first set of machine learning models 233), a set of analysis results, and identify, based on the set of analysis results, a high-variance subset 232 from the original dataset that includes a first set of subjects associated with a first analysis result that achieves a variance threshold.

The partition unit 224 is a functional unit for dividing high-variance datasets into partitions. More particularly, the partition unit 224 may partition the first set of subjects of the high-variance subset 232 into a first data partition and a second data partition, and generate a third data partition that is a copy of the first data partition and a fourth data partition that is a copy of the second data partition. In embodiments, the first set of subjects of the high-variance subset 232 may be divided into data partitions of true positive, false positive, true negative, or false negative based on the relationship between the analysis results generated by the first set of machine learning models 233 and ground truth.

The modification unit 226 is a functional unit for modifying (e.g., adding noise to) certain data partitions generated by the partition unit 224. More particularly, the modification unit 226 may generate a modified third data partition by modifying the third data partition and a modified fourth data partition by modifying the fourth data partition.

The labeling unit 228 is a functional unit for assigning outcome labels to the partitions generated by the partition unit 224. In embodiments, the labeling unit 228 may assign outcome labels of true positive or false positive (or true negative or false negative) to the modified third data partition and the modified fourth data partition.

The result generation unit 229 is a functional unit for training machine learning models based on the data partitions generated by the partition unit, and using these machine learning models to generate analysis results. More particularly, the result generation unit 229 may train a second set of machine learning models (e.g., the second set of machine learning models 234) using the first data partition, the second data partition, the modified third data partition and the modified fourth data partition, generate, by processing the original dataset with the second set of machine learning models, a second analysis result, and generate a final analysis result by aggregating and classifying the first analysis result and the second analysis result.

The storage unit 230 is a unit for storing various data and information used in implementing the aspects of the present disclosure. The storage unit 230 may include a collection of hard disc drives, solid state drives, flash memory, cloud, storage, or the like. As illustrated in FIG. 2, the storage unit 230 may include an original dataset 231, a high-variance subset 232, a first set of machine learning models 233, and a second set of machine learning models 234.

The original dataset 231 is a dataset to be used as part of a machine learning task. The original dataset 231 may include a set of subjects to be classified into one or more categories based on features associated with each subject. Here, "subjects" refer to entities within the original dataset. As described herein, all or a portion of the original dataset 231 may include sets of subjects associated with small sample sizes (e.g., sets for which the measured number of data points fails to achieve a predetermined threshold).

As an example, the original dataset 231 may include a dataset related to life quantity information and medical intervention information for patients, and can be used for a machine learning task of classifying patients (i.e., subjects) into groups based on their length of life likelihood as determined using features of patient characteristics or demographics (e.g., age, gender, race, income level, social determinants of health), laboratory results (e.g., hemoglobin, albumin, bilirubin, creatinine, sodium, alkaline phosphatase etc.), diagnosis results (e.g., renal failure, diabetes, cancer, heart failure, hypertension etc.) medical intervention history (past therapies, medications etc.), and the like.

As another example, the original dataset 231 may include a dataset related to equipment maintenance, and could be used for a machine learning task of classifying machine components (i.e., subjects) into groups based on their likelihood of failure as determined using features of machine component characteristics (e.g., device type, material, manufacturing data, age), device measurement or monitoring results (e.g., temperature, humidity, voltage, pressure), diagnosis results (e.g., voltage or current spikes, structural damage due to high pressure, overheating), maintenance history (e.g., past inspections, component replacements, software updates), and the like.

It should be noted that the original dataset 231 is not particularly limited herein, and other types of data may also be used.

The high-variance subset 232 is a portion of the original dataset 231 that has been identified as being associated with high-variance. In embodiments, the high-variance subset 232 may include a first set of subjects selected from among an original dataset.

The first set of machine learning models 233 are a first group of machine learning models trained based on the original dataset 231.

The second set of machine learning models 234 are a second group of machine learning models trained based on a modified training dataset generated based on the high-variance subset 232.

The processor 244 is a processing unit for executing processing instructions for the various software modules and functional units included in the information processing application 150 stored in the memory 220.

The input/output unit 246 is a unit for facilitating communication between the information processing device 210 and external sources, such as the user terminal 260. In embodiments, the input/output unit 246 may be configured to generate and provide a graphical user interface on the user terminal 260 to facilitate the input of data and user commands, and display status information and results.

The user terminal 260 is a device that can be used by a user (e.g., a client) of the information processing device 210. In embodiments, the user terminal 260 may be used to request analysis by the information processing device 210 of a set of data (e.g., the original dataset 231), and confirm the results of this analysis. As examples, the user terminal 260 may be implemented using a personal computer, tablet computer, smart phone, or other computing device.

According to the information processing system 200 illustrated in FIG. 2, it is possible to provide an information processing technique for generating machine learning models with a high degree of robustness that are capable of producing reliable results for data sets having small sample sizes.

As described herein, aspects of the present disclosure relate to the recognition that when prediction models are used to process datasets based on small sample sizes, the resulting analysis results may be associated with high variance between outcomes. Here, variance refers to the degree of error or uncertainty associated with a particular analysis result. Next, with reference to FIG. 3 and FIG. 4, examples of outcome variance will be described with reference to sample analysis results.

FIG. 3 is a diagram illustrating an example of a low-variance analysis result 310 and a high-variance analysis result 320 pertaining to the survival rate of patients with respect to a particular medical intervention. In the low-variance analysis result 310 and the high-variance analysis result 320, the vertical axis represents the survival rate (in percent) and the horizontal axis represents the time lived after a medical intervention. As illustrated in FIG. 3, the line plot 312 of the low-variance analysis result 310 is associated with a variance range 315, and the line plot 322 of the high-variance analysis result 320 is associated with a variance range 325. These variance ranges 315 and 325 indicate the range of error in the survival rate of the patients as a function of the number of years that have passed since medical intervention.

As can be seen by comparing the low-variance analysis result 310 and the high-variance analysis result 320, whereas the variance range 315 of the low-variance analysis result 310 is less than 10%, the variance range 325 of the high-variance analysis result is nearly 60%. As described herein, due to this high variance between different outcomes, results such as the high-variance analysis result are unreliable, and are not suitable for drawing conclusions or insights.

Accordingly, aspects of the disclosure relate to generating machine learning models with a high degree of robustness that are capable of producing reliable results for data sets having small sample sizes.

FIG. 4 is a diagram illustrating an example of a reduced-variance analysis result 410 according to the embodiments of the present disclosure. The reduced-variance analysis result 410 indicates an analysis result generated by using the information processing technique according to the embodiments of the present disclosure with respect to the same dataset used to create the high-variance analysis result 320 illustrated in FIG. 3.

As described with reference to FIG. 3, in the reduced-variance analysis result 410, the vertical axis represents the survival rate (in percent) and the horizontal axis represents the time lived after a medical intervention. As illustrated in FIG. 4, the line plot 412 of the reduced-variance analysis result 410 is associated with a variance range 415. This variance ranges 415 indicates the range of error in the survival rate of the patients as a function of the number of years that have passed since medical intervention.

As can be seen by comparing the reduced-variance analysis result 410 and the high-variance analysis result 320 illustrated in FIG. 3, the variance range 415 of the reduced-variance analysis result 410 has been reduced by approximately 10% with respect to the high-variance analysis result 320. Due to this reduced variance range, the reduced-variance analysis result 410 can be used to facilitate the drawing of more accurate conclusions or insights than the high-variance analysis result 320.

Next, with reference to FIG. 5, the information processing method according to the embodiments of the present disclosure will be described.

FIG. 5 is a flowchart illustrating an information processing method 500 according to the embodiments of the present disclosure. The information processing method 500 illustrated in FIG. 5 is a method for identifying datasets associated with high-variance analysis results, generating machine learning models with a high degree of robustness, and generating an analysis result for datasets having small sample sizes. The information processing method 500 may be performed by the various functional units of the information processing device 210 illustrated in FIG. 2.

First, at Step S505, the variance analysis unit 222 generates a set of analysis results by processing an original dataset (e.g., the original dataset 231 illustrated in FIG. 2) using a first set of machine learning models (e.g., the first set of machine learning models 233 illustrated in FIG. 2). The first set of machine learning models may be machine learning models trained to perform a classification task with respect to the original dataset. As described herein, the original dataset may be a dataset designated for use as part of a machine learning classification task. For instance, the original dataset may include a set of subjects to be classified into one or more categories based on features associated with each subject. Here, "subjects" refer to entities within the original dataset. As an example, the original dataset may include personal life quantity, medical characteristics, and medical intervention information for patients (e.g., subjects). As described herein, all or a portion of the original dataset's subjects may be associated with small sample sizes. For instance, patients associated with rare medical interventions may be associated with small sample sizes.

The first set of machine learning models may process all or a portion of the original dataset to generate a set of analysis results in which the set of subjects of the original dataset are classified into categories. This set of analysis results may include a first analysis result corresponding to a first set of subjects of the original dataset.

As an example, in the case that the original dataset includes personal medical characteristics and medical intervention information for patients, the first set of machine learning models may generate a set of analysis results in which each patient is sorted into a category based on their survival likelihood for particular medical interventions.

Next, at Step S510, the variance analysis unit 222 identifies, based on the set of analysis results generated in Step S505, a high-variance subset (e.g., the high-variance subset 232 illustrated in FIG. 2) including a first set of subjects from among set of subjects in the original dataset that are associated with a first analysis result that achieves a variance threshold. As described herein, variance refers to the degree of error or uncertainty associated with a particular analysis result. This variance may arise due to the sensitivity of the first set of machine learning models to small fluctuations in the original dataset, and may be higher in cases in which the sample size is relatively small. Additionally, the variance threshold refers to a criterion or standard that defines a boundary between high-variance and low-variance outcomes with respect to the set of analysis results. In embodiments, the variance threshold may be determined based on the nature of the machine learning task, and may be defined in terms of a number of standard deviations, a percentage of, or the like. As an example, in the case that the variance threshold is "30%," those subjects associated with analysis results having 30% or greater variance between outcomes may be identified as achieving the variance threshold.

Next, at Step S515, the partition unit 224 partitions the first set of subjects of the high-variance subset 232 identified in Step S510 into two or more data partitions. In embodiments, the partition unit 224 may partition the first set of subjects of the high-variance subset 232 based on an accuracy factor of the first analysis result generated for the first set of subjects in Step S505 by comparing the first analysis result with respect to a ground truth result of the original dataset. More particularly, for each subject of the first set of subjects, the partition unit 224 may compare the result indicated for that subject in the first analysis result with a ground truth result associated with the original dataset that indicates the actual, correct outcome for that subject, and based on the relationship between the first analysis result and the ground truth result, sort the first set of subjects into different partitions associated with true-positive results, false-positive results, true-negative results and false-negative results. Herein, for convenience of description, an example configuration will be described for a case in which a first subset of the first set of subjects associated with a true-positive result are placed in a first data partition, and a second subset of the first set of subjects associated with a false-positive result are placed in a second data partition, but the present disclosure is not limited herein, and a configuration in which a third subset of the first set of subjects associated with a true-negative result are placed in a third data partition and a fourth subset of the first set of subjects associated with a false-negative result are placed in a fourth data partition are also possible.

In this way, by dividing the high-variance subset 232 into partitions of true-positive results, false-positive results, true-negative results and false-negative results, it becomes possible to individually manage those subjects for which machine learning classification performance is higher separately from those subjects for which machine learning classification performance is lower. As a result, as will be described later, different amounts of noise can be added to particular partitions.

Next, at Step S520, the partition unit 224 generates a copy of each data partition created in Step S515. More particularly, in the case that a first data partition associated with true-positive results and a second data partition associated with false-positive results were created in Step S515, the partition unit 224 may create a third data partition that is a copy of the first data partition and a fourth data partition that is a copy of the second data partition.

Next, at Step S525, the modification unit 226 modifies the copied data partitions created in Step S515 to create modified data partitions. Here, the modification unit 226 may modify the copied data partitions by adding noise to a set of features of each data partition to distort or alter the values of those features. Different amounts of noise may be added to each partition. For instance, the modification unit 226 may generate a modified third data partition by adding a first set of noise to a third set of features of the third data partition and generate a modified fourth data partition by adding a second set of noise to a fourth set of features of the fourth data partition. The amount of noise added to the set of features of the copied data may be determined based on a noise level criterion. As will be described later, this noise level criterion may be determined based on the performance of a second set of machine learning models with respect to sample datasets with varying amounts of noise. In addition, as will be described later, the modified third data partition and the modified fourth data partition may be assigned labels by the labeling unit 228.

Next, at Step S530, the result generation unit 229 trains a second set of machine learning models (e.g., the second set of machine learning models 234 illustrated in FIG. 2) using the first data partition and the second data partition generated in Step S515 and the modified third data partition and the modified fourth data partition generated in Step S525. Here, the second set of machine learning models are untrained machine learning models that are of the same type and configuration as the first set of machine learning models. By training the second set of machine learning models using the first and second data partitions that contain data from the original dataset together with modified third and fourth data partitions that have been modified to have distorted features, the robustness of the second set of machine learning models with respect to small sample sizes can be increased.

Next, at Step S535, the result generation unit 229 generates a second analysis result by processing the first set of subjects of the high-variance subset 232 identified in Step S510 using the second set of machine learning models trained in Step S530. In embodiments, the second set of machine learning models may be configured to perform the same machine learning task on the same data as in Step S505. In this way, in addition to the first analysis result generated for the first set of subjects by the first set of machine learning models, a second analysis result for the same first set of subjects can be generated by the second set of machine learning models that have been trained to have increased robustness with respect to small sample sizes.

Next, at Step S540, the result generation unit 229 may generate a final analysis result by aggregating the first analysis result generated in Step S505 and the second analysis result generated in Step S535, and classifying (e.g., re-classifying and re-stratifying) the results. Here, the first analysis result and the second analysis result may be aggregated using any conventional machine learning result aggregation technique, and the results are not particularly limited herein. Accordingly, by combining the first analysis result generated for the first set of subjects together with the second analysis result for the same first set of subjects that has been generated by the second set of machine learning models trained to have increased robustness with respect to small sample sizes, it is possible to acquire a more accurate final analysis result that has increased accuracy with respect to the first analysis result.

According to the information processing method 500, it is possible to provide an information processing technique for generating machine learning models with a high degree of robustness that are capable of producing reliable results for data sets having small sample sizes. More particularly, as the second set of machine learning models is trained based on a modified version of the original dataset in which the features have been distorted with noise, this second set of machine learning models can be trained to recognize those features that are most salient to the classification task, and robust with respect to small sample sizes. In this way, it becomes possible to acquire reliable, highly-accurate machine learning analysis results even in small sample sizes for which conventional machine learning techniques would yield high-variance results.

Next, with reference to FIG. 6, an example logical configuration of an information processing device for identifying datasets associated with high variance results will be described.

FIG. 6 is a diagram illustrating an example logical configuration of the information processing device 210 for identifying datasets associated with high variance results, according to the embodiments of the present disclosure. In embodiments, the functions performed by the information processing device 210 to identify datasets associated with high variance results may substantially correspond to Steps S505-S510 of the information processing method 500 illustrated in FIG. 5. In addition, the functions performed by the information processing device 210 to identify datasets associated with high variance results may be performed by the variance analysis unit 222 (not illustrated in FIG. 6).

First, the variance analysis unit 222 acquires the original dataset 231. As described herein, the original dataset 231 may be a dataset designated for use as part of a machine learning classification task. For instance, the original dataset 231 may include a set of subjects to be classified into one or more categories based on features associated with each subject. In embodiments, the original dataset 231 may be created and designated by a user for use as part of a machine learning classification task.

Next, the variance analysis unit 222 generates a machine learning dataset 605 by performing feature selection 602 on the original dataset 231. More particularly, the variance analysis unit 222 may identify a set of salient features within the original dataset that are determined to be relevant to the machine learning task to be performed, and generate the machine learning dataset 605 by extracting the subjects and the corresponding salient features for those subjects. In embodiments, the set of salient features may be determined in advance by the user for a given machine learning task. In general, the more salient features that are selected for inclusion in the machine learning dataset 605, the greater the accuracy that can be achieved by the machine learning models; however, more salient features in the machine learning dataset 605 also increases the computing resources required for performance of the machine learning task. Accordingly, the type and number of salient features for inclusion in the machine learning dataset may be selected by a user or administrator based on the nature of the machine learning task.

Next, the variance analysis unit 222 performs partition generation 604 on the machine learning dataset 605 to divide the machine learning dataset 605 into a set of development partitions 606 and a set of evaluation partitions 608. In embodiments, the set of development partitions 606 and the set of evaluation partitions 608 may be mutually exclusive with respect to each other; that is, none of the data included in the set of development partitions 606 is included in the set of evaluation partitions 608, and none of the data included in the set of evaluation partitions 608 is included in the set of development partitions 606. Further, each individual partition of the set of development partitions 606 and each individual partition of the set of evaluation partitions may be mutually exclusive with respect to the other individual partitions of the set. In certain embodiments, each individual partition of the set of development partitions 606 and each individual partition of the set of evaluation partitions 608 may include a single subject and its associated features. In certain embodiments, each individual partition of the set of development partitions 606 and each individual partition of the set of evaluation partitions may include multiple subjects and their associated features.

In embodiments, the variance analysis unit 222 may randomly divide the machine learning dataset 605 into the set of development partitions 606 and the set of evaluation partitions 608. This partitioning may be based on a predetermined proportion. For instance, a randomly selected assortment of data comprising 80% of the data of the machine learning dataset 605 may be used as the set of development partitions 606 and the remaining 20% of the data of the machine learning dataset may be used as the set of evaluation partitions 608.

Next, the variance analysis unit 222 may use the set of development partitions 606 to train a first set of machine learning models 233 to perform a classification task (e.g., risk classification of patient survivability, component failure, or the like). Here, the first set of machine learning models 233 may be a machine learning model ensemble including a plurality of untrained predictive models. In embodiments, the variance analysis unit 222 may train each individual machine learning model of the first set of machine learning models 233 using a separate partition of the set of development partitions 606. Here, the first set of machine learning models 233 may be trained using existing training methods, and the training method is not particularly limited herein. In this way, the first set of machine learning models 233 may be trained to perform a classification task using the set of development partitions 606.

Next, the variance analysis unit 222 may use the trained first set of machine learning models 233 to perform a classification task on the set of evaluation partitions 608. Each machine learning model of the set of machine learning models 233 may process a separate partition of the set of evaluation partitions 608 to generate a set of analysis results 620. In embodiments, an analysis result may be generated for each subject included in the set of evaluation partitions 608. The set of analysis results 620 may indicate the likelihood of a subject corresponding to a particular category or phenomenon. Subjects may be assigned classification scores (e.g., risk scores) and clustered into groups (e.g., risk groups) based on these calculated likelihoods. Further, as described herein, each analysis result of the generated set of analysis results 620 may be associated with a variance range indicating the uncertainty in the outcome of that analysis result for that particular subject.

Next, the variance analysis unit 222 may analyze the set of analysis results 620 to identify a high-variance subset of the original dataset 231 that includes first set of subjects associated with a first analysis result 625 that achieves a variance threshold. Here, the variance threshold refers to a criterion or standard that defines a boundary between high-variance and low-variance outcomes with respect to the set of analysis results. In embodiments, the variance threshold may be determined based on the nature of the machine learning task, and may be defined in terms of a number of standard deviations, a percentage of, or the like. As an example, in the case that the variance threshold is "30%," those subjects associated with analysis results having a variance range of 30% or greater may be identified as achieving the variance threshold.

In this way, according to the variance analysis unit 222 according to the embodiments of the present disclosure, it is possible to identify those subjects that are associated with high variance results. As described herein, these high-variance subjects (e.g., the first set of subjects and the associated first analysis result 625) may be used to facilitate the generation of machine learning models with a high degree of robustness that are capable of producing reliable results for data sets having small sample sizes.

Next, with reference to FIG. 7, an example logical configuration of an information processing device 210 for generating analysis results for high-variance datasets will be described.

FIG. 7 is a diagram illustrating an example logical configuration of an information processing system 200 for generating analysis results for high-variance datasets, according to the embodiments of the present disclosure. In embodiments, the functions performed by the information processing device 210 to generate analysis results for high-variance datasets may substantially correspond to Steps S515-S540 of the information processing method 500 illustrated in FIG. 5. In addition, the functions performed by the information processing device 210 to generate analysis results for high-variance datasets may be performed by the partition unit 224, the modification unit 226, the labeling unit 228 and the result generation unit 229 after subjects associated with high-variance results are identified by the functions of the variance analysis unit 222 as described with reference to FIG. 6.

First, the partition unit 224 partitions the first set of subjects of the high-variance subset 232 identified by the variance analysis unit 222 (described with reference to FIG. 6) into a first data partition 707 and a second data partition 709. Here, the partition unit 224 may partition the first set of subjects of the high-variance subset 232 based on an accuracy factor of a first analysis result 625 generated for the first set of subjects of the high-variance subset 232 with respect to a ground truth result corresponding to the first set of subjects. More particularly, for each subject of the first set of subjects in the high-variance subset 232, the partition unit 224 may compare the result indicated for that subject by the first analysis result 625 with a ground truth result associated with the first set of subjects that indicates the actual, correct outcome for that subject, and based on the relationship between the first analysis result 625 and the ground truth result, sort the first set of subjects of the high-variance subset 232 into different partitions associated with true-positive results, false-positive results, true-negative results and false-negative results.

Herein, for convenience of description, an example configuration will be described for a case in which a first subset of the first set of subjects associated with a true-positive result are placed in a first data partition 707, and a second subset of the first set of subjects associated with a false-positive result are placed in a second data partition 709, but the present disclosure is not limited herein, and a configuration in which a third subset of the first set of subjects associated with a true-negative result are placed in a third data partition and a fourth subset of the first set of subjects associated with a false-negative result are placed in a fourth data partition are also possible.

Next, the partition unit 224 generates a copy of the first data partition 707 and the second data partition 709. More particularly, the partition unit 224 may create a third data partition 708 that is a copy of the first data partition 707 and a fourth data partition 710 that is a copy of the second data partition 709.

Next, the modification unit 226 modifies the third data partition 708 to create a modified third data partition 711 and modifies the fourth data partition 710 to create a modified fourth data partition 712. Here, the modification unit 226 may modify the third data partition 708 and the fourth data partition 710 by adding noise to a set of features of each data partition to distort or alter the values of those features. The amount of noise added to the set of features of the copied data may be determined based on a noise level criterion.

The noise level criterion is information that indicates the desirable amount of noise to be added to the set of features of each data partition. Here, "the desirable amount of noise" refers to the maximum amount of noise that can be added to the features of a particular subject without resulting in the result label (e.g., true-positive, false-positive, true-negative, false-negative) for that subject changing. This is because, by adding more noise to the set of features while still maintaining the same result, the second set of machine learning models 234 to be trained using these modified datasets can become more robust with respect to noise while still recognizing the salient features of the dataset that correspond to a particular analysis result.

In embodiments, the modification unit 226 may be configured to use a machine learning model (e.g., the second set of machine learning models 234 or a separate test set of machine learning models) to consecutively process modified sample datasets in which increasingly greater amounts of noise have been added to the features, and identify the maximum amount of noise that can be added to the features such that the sample prediction results generated for the modified sample dataset satisfies a predetermined accuracy threshold (e.g., a minimum accuracy threshold). In this way, the maximum amount of noise that can be added to the features while maintaining a desired accuracy threshold can be identified and set as the noise level criterion.

Further, based on this noise level criterion, different amounts of noise may be added to each data partition. As an example, a greater amount of noise may be added to the third data partition 708 associated with true-positive results, and a lesser amount of noise may be added to the fourth data partition 710 associated with false-positive results. In this way, the appropriate amount of noise necessary to suitably obscure the features of each data partition may be separately applied to each data partition.

Next, the labeling unit 228 may assign outcome labels to the modified third data partition 711 and the modified fourth data partition 712. Here, the outcome labels refer to the predicted relationship between an analysis result generated based on the modified third data partition 711 and the modified fourth data partition 712 (e.g., true-positive, false-positive, true-negative, false-negative) and the ground truth result. As the analysis results for the modified third data partition 711 and the modified fourth data partition 712 should theoretically be same as those of the data partitions from which they were created (e.g., the first data partition 707 and the second data partition 709), in embodiments, the labeling unit 228 may assign true-positive outcome labels to the modified third data partition 711 and assign false positive outcome labels to the modified fourth data partition 712.

However, aspects of the present disclosure relate to the recognition that, due to the noise added to the data partitions 707, 709, the outcomes generated for the modified data partitions 711, 712 are not guaranteed to correspond to those of the data partitions 707, 709. Accordingly, in embodiments, the labeling unit 228 may calculate the similarity between the modified data partitions 711, 712 and the original data partitions 707, 709, and assign outcome labels to the modified data partitions 711, 712 based on the similarity of the modified data partitions 711, 712 to the original data partitions 707, 709.

More particularly, the labeling unit 228 may calculate a first similarity factor between a first subset of subjects of the first data partition 707 and a third subset of subjects of the modified third data partition 711, assign a true-positive result label to the third subset of subjects in the case that the first similarity factor achieves a similarity threshold, and assign a false-positive result label to the third subset of subjects in the case that the first similarity factor does not achieve a similarity threshold. Similarly, the labeling unit 228 may calculate a second similarity factor between a second subset of subjects of the second data partition 709 and a fourth subset of subjects of the modified fourth data partition 712, assign a false-positive result label to the fourth subset of subjects in a case that the second similarity factor achieves a similarity threshold, and assign a true-positive result label to the fourth subset of subjects in a case that the second similarity factor does not achieve a similarity threshold.

In this way, more accurate outcome labels can be assigned to the modified data partitions 711, 712, and training of a second set of machine learning models 234 can be facilitated. Here, the similarity between the modified data partitions 711, 712 and the original data partitions 707, 709 may be calculated using Euclidean distance, cosine similarity, or other conventional similarity algorithm.

Next, the result generation unit 229 trains a second set of machine learning models 234 (e.g., the second set of machine learning models 234 illustrated in FIG. 2) using a modified training dataset 713 that includes the first data partition 707, the second data partition 709, the modified third data partition 711 and the modified fourth data partition 712. Here, the second set of machine learning models 234 are untrained machine learning models that are of the same type and configuration as the first set of machine learning models. By training the second set of machine learning models 234 using the first and second data partitions 707, 709 that contain data from the original dataset together with modified third and fourth data partitions 711, 712 that have been modified to have distorted features, the robustness of the second set of machine learning models 234 with respect to small sample sizes can be increased.

Next, the result generation unit 229 generates a second analysis result 715 by processing the first set of subjects of the high-variance subset 232 using the second set of machine learning models 234. In embodiments, the second set of machine learning models 234 may be configured to perform the same machine learning task on the first set of subjects of the high-variance subset 232 that the first set of machine learning models 233 performed on the set of evaluation partitions 608 described in FIG. 6.

Next, the result generation unit 229 may generate a final analysis result 720 by aggregating the first analysis result 625 generated by the first set of machine learning models 233 and the second analysis result 715 generated by the second set of machine learning models 234, and classifying (e.g., re-classifying and re-stratifying) the results. Here, the first analysis result and the second analysis result may be aggregated using any conventional machine learning result aggregation technique, and the results are not particularly limited herein.

According to the functions of the information processing system 200 described with reference to FIG. 7, the second set of machine learning models are trained on a modified training dataset that includes an original high-variance dataset together with a version of the high-variance dataset that has been altered with noise. In this way, the second set of machine learning models can be trained to become robust with respect to datasets with small sample sizes, and machine learning analysis results with reduced outcome variance can be generated.

As described herein, in machine learning, it is preferable that the accuracy of a generated model, that is, the ability to accurately predict the result of an unknown case (sometimes called prediction performance) is high. The prediction performance usually increases as the size of the dataset (i.e., the sample size), increases. Conversely, datasets based on small sample sizes can lead to decreased prediction performance, such as results with large variability in outcomes. Due to this high variance between different outcomes, such results may be unreliable, and are not suitable for drawing conclusions or insights.

In embodiments, aspects of the present disclosure relate to using a first set of machine learning models to generate a set of analysis results for an original dataset, and use this set of analysis results to identify a subset of the original dataset that achieves a variance threshold (e.g., has a high level of variance between outcomes). In this way, subsets of the original dataset that are associated with high-variance can be identified.

Additional aspects of the present disclosure relate to partitioning the identified high-variance subset into individual data partitions. These data partitions may classify portions of the high-variance subset into categories of true positive, false positive, true negative, or false negative based on the relationship between the analysis results generated by the first set of machine learning models and ground truth. By partitioning the high-variance subset into partitions of true positive, false positive, true negative, and false negative, the dataset portions for which machine learning classification performance is higher can be managed separately from the dataset portions for which machine learning classification performance is lower. Each generated partition may be copied, and noise may be added to each copied data partition. The noise added to each copied data partition may be determined individually for each partition. The copied data partitions to which noise has been added may be merged with the original data partitions to generate a modified training dataset.

This modified dataset can be used to train a second set of machine learning models. In this way, the second set of machine learning models can be trained to become robust with respect to datasets with small sample sizes. The trained second set of machine learning models can be used to generate an analysis result (e.g., a second analysis result) for the high-variance subset extracted from the original dataset, and this analysis result can be aggregated with the analysis result generated by the first set of machine learning results to generate a final analysis result with reduced outcome variance.

Accordingly, it is possible to provide an information processing technique for generating machine learning models with a high degree of robustness that are capable of producing reliable results for data sets having small sample sizes. More particularly, as the second set of machine learning models is trained based on a modified version of the original dataset in which the features have been distorted with noise, this second set of machine learning models can be trained to recognize those features that are most salient to the classification task, and robust with respect to small sample sizes. In this way, it becomes possible to acquire reliable, highly-accurate machine learning analysis results even in small sample sizes for which conventional machine learning techniques would yield high-variance results.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing.

A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

While the foregoing is directed to exemplary embodiments, other and further embodiments of the invention may be devised without departing from the basic scope thereof, and the scope thereof is determined by the claims that follow. The descriptions of the various embodiments of the present disclosure have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to explain the principles of the embodiments, the practical application or technical improvement over technologies found in the marketplace, or to enable others of ordinary skill in the art to understand the embodiments disclosed herein.

The terminology used herein is for the purpose of describing particular embodiments only and is not intended to be limiting of the various embodiments. As used herein, the singular forms "a," "an," and "the" are intended to include the plural forms as well, unless the context clearly indicates otherwise. "Set of," "group of," "bunch of," etc. are intended to include one or more. It will be further understood that the terms "includes" and/or "including," when used in this specification, specify the presence of the stated features, integers, steps, operations, elements, and/or components, but do not preclude the presence or addition of one or more other features, integers, steps, operations, elements, components, and/or groups thereof. In the previous detailed description of exemplary embodiments of the various embodiments, reference was made to the accompanying drawings (where like numbers represent like elements), which form a part hereof, and in which is shown by way of illustration specific exemplary embodiments in which the various embodiments may be practiced. These embodiments were described in sufficient detail to enable those skilled in the art to practice the embodiments, but other embodiments may be used and logical, mechanical, electrical, and other changes may be made without departing from the scope of the various embodiments. In the previous description, numerous specific details were set forth to provide a thorough understanding the various embodiments. But, the various embodiments may be practiced without these specific details. In other instances, well-known circuits, structures, and techniques have not been shown in detail in order not to obscure embodiments.

As described herein, aspects of the present disclosure relate to the following aspects.

(Aspect 1) An information processing device comprising:
a processor; and
a memory containing computer implementable instructions executable by the processor to cause the processor to function as:
   a variance analysis unit configured to:
      generate, by processing an original dataset with a first set of machine learning models, a set of analysis results, and
      identify, based on the set of analysis results, a high-variance subset from the original dataset that includes a first set of subjects associated with a first analysis result that achieves a variance threshold;
   a partition unit configured to:
      partition the first set of subjects of the high-variance subset into a first data partition and a second data partition, and
      generate a third data partition that is a copy of the first data partition and a fourth data partition that is a copy of the second data partition,
   a modification unit configured to generate a modified third data partition by modifying the third data partition and a modified fourth data partition by modifying the fourth data partition; and
   a result generation unit configured to:
      train a second set of machine learning models using the first data partition, the second data partition, the modified third data partition and the modified fourth data partition,
      generate, by processing the original dataset with the second set of machine learning models, a second analysis result, and
      generate a final analysis result for the first set of subjects of the high-variance subset by aggregating and classifying the first analysis result and the second analysis result.

(Aspect 2) The information processing device according to aspect 1, wherein the modification unit is configured to:
generate the modified third data partition by adding, based on a noise level criterion, noise to a third set of features of the third data partition; and
generate the modified fourth data partition by adding, based on the noise level criterion, noise to a fourth set of features of the fourth data partition.

(Aspect 3) The information processing device according to aspect 2, wherein the modification unit is configured to:
generate, by using the second set of machine learning models to iteratively process modified sample datasets to which increasing amounts of noise have been added, a set of sample prediction results;
identify, from among the set of sample prediction results, a subset of sample prediction results that satisfy a predetermined accuracy threshold; and
determine, as the noise level criterion, a maximum amount of noise that was added to the modified sample datasets associated with the subset of sample prediction results that satisfy the predetermined accuracy threshold.

(Aspect 4) The information processing device according to any one of aspects 1 to 3, wherein the partition unit is configured to:
evaluate an accuracy factor of the first analysis result by comparing the first analysis result with respect to a ground-truth result for the original dataset;
partition a first subset of the first set of subjects associated with a true-positive result into the first data partition; and
partition a second subset of the first set of subjects associated with a false positive result into the second data partition.

(Aspect 5) The information processing device according to any one of aspects 1 to 4, further comprising:
a labeling unit for assigning outcome labels of true positive or false positive to the modified third data partition and the modified fourth data partition.

(Aspect 6) The information processing device according to aspect 5, wherein the label unit:
calculates a first similarity factor between a first subset of subjects of the first data partition and a third subset of subjects of the modified third data partition;
assigns, in a case that the first similarity factor achieves a similarity threshold, a true-positive result label to the third subset of subjects;
assigns, in a case that the first similarity factor does not achieve a similarity threshold, a false-positive result label to the third subset of subjects;
calculates a second similarity factor between a second subset of subjects of the second data partition and a fourth subset of subjects of the modified fourth data partition;
assigns, in a case that the second similarity factor achieves a similarity threshold, a false-positive result label to the fourth subset of subjects; and
assigns, in a case that the second similarity factor does not achieve a similarity threshold, a true-positive result label to the fourth subset of subjects.

### [Reference Signs List]

- 150: Information Processing Application
- 200: Information Processing System
- 210: Information Processing Device
- 220: Memory
- 222: Variance Analysis Unit
- 224: Partition Unit
- 226: Modification Unit
- 228: Labeling Unit
- 229: Result Generation Unit
- 230: Storage Unit
- 231: Original Dataset
- 232: High-variance subset
- 233: First Set of Machine Learning Models
- 234: Second Set of Machine Learning Models
- 244: Processor
- 246: Input/Output Unit
- 250: Communication Network
- 260: User Terminal

## Claims

1. An information processing device comprising:
a processor; and
a memory containing computer implementable instructions executable by the processor to cause the processor to function as:
a variance analysis unit configured to:
generate, by processing an original dataset with a first set of machine learning models, a set of analysis results, and
identify, based on the set of analysis results, a high-variance subset from the original dataset that includes a first set of subjects associated with a first analysis result that achieves a variance threshold;
a partition unit configured to:
partition the first set of subjects of the high-variance subset into a first data partition and a second data partition, and
generate a third data partition that is a copy of the first data partition and a fourth data partition that is a copy of the second data partition,
a modification unit configured to generate a modified third data partition by modifying the third data partition and a modified fourth data partition by modifying the fourth data partition; and
a result generation unit configured to:
train a second set of machine learning models using the first data partition, the second data partition, the modified third data partition and the modified fourth data partition,
generate, by processing the original dataset with the second set of machine learning models, a second analysis result, and
generate a final analysis result for the first set of subjects of the high-variance subset by aggregating and classifying the first analysis result and the second analysis result.

2. The information processing device according to claim 1, wherein the modification unit is configured to:
generate the modified third data partition by adding, based on a noise level criterion, a first amount of noise to a third set of features of the third data partition; and
generate the modified fourth data partition by adding, based on the noise level criterion, a second amount of noise to a fourth set of features of the fourth data partition.

3. The information processing device according to claim 2, wherein the modification unit is configured to:
generate, by using the second set of machine learning models to iteratively process modified sample datasets to which increasing amounts of noise have been added, a set of sample prediction results;
identify, from among the set of sample prediction results, a subset of sample prediction results that satisfy a predetermined accuracy threshold; and
determine, as the noise level criterion, a maximum amount of noise that was added to the modified sample datasets associated with the subset of sample prediction results that satisfy the predetermined accuracy threshold.

4. The information processing device according to claim 1, wherein the partition unit is configured to:
evaluate an accuracy factor of the first analysis result by comparing the first analysis result with respect to a ground-truth result for the original dataset;
partition a first subset of the first set of subjects associated with a true-positive result into the first data partition; and
partition a second subset of the first set of subjects associated with a false positive result into the second data partition.

5. The information processing device according to claim 1, further comprising:
a labeling unit for assigning outcome labels of true positive or false positive to the modified third data partition and the modified fourth data partition.

6. The information processing device according to claim 5, wherein the label unit:
calculates a first similarity factor between a first subset of subjects of the first data partition and a third subset of subjects of the modified third data partition;
assigns, in a case that the first similarity factor achieves a similarity threshold, a true-positive result label to the third subset of subjects;
assigns, in a case that the first similarity factor does not achieve a similarity threshold, a false-positive result label to the third subset of subjects;
calculates a second similarity factor between a second subset of subjects of the second data partition and a fourth subset of subjects of the modified fourth data partition;
assigns, in a case that the second similarity factor achieves a similarity threshold, a false-positive result label to the fourth subset of subjects; and
assigns, in a case that the second similarity factor does not achieve a similarity threshold, a true-positive result label to the fourth subset of subjects.

7. An information processing system comprising:
an information processing device; and
a user terminal;
wherein the information processing device includes:
a processor; and
a memory containing computer implementable instructions executable by the processor to cause the processor to function as:
a variance analysis unit configured to:
generate, by processing an original dataset with a first set of machine learning models, a set of analysis results, and
identify, based on the set of analysis results, a high-variance subset from the original dataset that includes a first set of subjects associated with a first analysis result that achieves a variance threshold;
a partition unit configured to:
partition the first set of subjects of the high-variance subset into a first data partition and a second data partition, and
generate a third data partition that is a copy of the first data partition and a fourth data partition that is a copy of the second data partition,
a modification unit configured to generate a modified third data partition by modifying the third data partition and a modified fourth data partition by modifying the fourth data partition; and
a result generation unit configured to:
train a second set of machine learning models using the first data partition, the second data partition, the modified third data partition and the modified fourth data partition,
generate, by processing the original dataset with the second set of machine learning models, a second analysis result,
generate a final analysis result for the first set of subjects of the high-variance subset by aggregating and classifying the first analysis result and the second analysis result; and
output the final analysis result to the user terminal.

8. An information processing method carried out by a computer, the information processing method comprising steps of:
generating, by processing an original dataset with a first set of machine learning models, a set of analysis results;
identifying, based on the set of analysis results, a high-variance subset from the original dataset that includes a first set of subjects associated with a first analysis result that achieves a variance threshold;
partitioning the first set of subjects of the high-variance subset into a first data partition and a second data partition;
generating a third data partition that is a copy of the first data partition and a fourth data partition that is a copy of the second data partition;
generating, by using a test set of machine learning models to iteratively process modified sample datasets to which increasing amounts of noise have been added, a set of sample prediction results;
identifying, from among the set of sample prediction results, a subset of sample prediction results that satisfy a predetermined accuracy threshold;
determining, as a noise level criterion, a maximum amount of noise that was added to the modified sample datasets associated with the subset of sample prediction results that satisfy the predetermined accuracy threshold;
generating, by adding a first amount of noise to a third set of features of the third data partition based on the noise level criterion, a modified third data partition;
generating, by adding a second amount of noise a fourth set of features of the fourth data partition based on the noise level criterion, a modified fourth data partition;
training a second set of machine learning models using the first data partition, the second data partition, the modified third data partition and the modified fourth data partition;
generating, by processing the original dataset with the second set of machine learning models, a second analysis result; and
generating a final analysis result for the first set of subjects of the high-variance subset by aggregating and classifying the first analysis result and the second analysis result.
